# EUROPEAN PATENT APPLICATION

(11) **EP 3 381 822 A1**
(43) Date of publication of application: **03.10.2018**
(21) Application number: 17164190.5
(22) Date of filing: 31.03.2017
(51) Int. Cl.: B65C 3/10, B65C 9/26, A61B 90/90

(54) **LABEL FIXATION FOR MEDICAL INSTRUMENTS**

(71) Applicant: Koninklijke Philips N.V., 5656 AE Eindhoven (NL)
(72) Inventor: DRIES, Johan Juliana, 5656 AE Eindhoven (NL); HOMAN, Robert Johannes Frederik, 5656 AE Eindhoven (NL); HOLTHUIZEN, IRonaldus Frederik Johannes, 5656 AE Eindhoven (NL)
(74) Representative: de Haan, Poul Erik

(57) **Abstract**

The present invention relates to a label fixation. In order to provide an improved labelling of medical instruments, a label fixation device (10) is provided. The label fixation device comprises a first reception (12) for temporarily receiving a longitudinal body portion of a medical instrument, and a second reception (14) for arranging a label to be fixed to a medical instrument. The first reception and the second reception are movable (16) in relation to each other for providing an abutting contact of a received longitudinal body portion of the medical instrument in the first reception and an arranged label in the second reception. The first reception provides a holding position for a medical instrument, in which holding position the longitudinal body portion is rotatably held around a longitudinal axis of the longitudinal body portion. The second reception provides a supply position for a label, in which supply position a front edge of the label is arranged aligned with the longitudinal axis of the longitudinal body portion.

## Description

### FIELD OF THE INVENTION

The present invention relates to a label fixation device, to a pattern application system for identifying or tracking medical instruments and to a method for label fixation to a medical instrument.

### BACKGROUND OF THE INVENTION

In relation with medical interventions, e.g. surgery and examination, a plurality of devices may be used, i.e. medical instruments such as surgical and other interventional instruments. For various reasons, such as for surgical navigation, device tracking is used to provide information about, e.g. the used or available devices. Devices can be tracked in different ways. For example, electromagnetic tracking can be provided throughout an operational environment, e.g. by RFID tags or the like. Devices, in particular surgical and interventional devices used when also X-ray imaging is provided, can also be tracked by identification of the devices in the X-ray images. Identification may be provided by labels on devices. For example, US 2009 114729 A1 describes a sterile medication identification delivery and application system. Another way of tracking devices is by optical recognition for example of a predefined pattern that is present on the device, such as on the shaft of the device. As an example, a pattern may be provided on a label and the label is attached to the device for optical identification. For a facilitated optical identification, simplified graphical patterns may be provided. However, it has been shown that such patterns may require an exact attachment on the device, which may cumbersome to achieve in particular during a surgical procedure when additional devices may need to be identified and tracked.

### SUMMARY OF THE INVENTION

There may thus be a need to provide an improved labelling of medical instruments.

The object of the present invention is solved by the subject-matter of the independent claims; further embodiments are incorporated in the dependent claims. It should be noted that the following described aspects of the invention apply for the label fixation device, and also for the position tracking and identification system for medical instruments and for the method for label fixation to a medical instrument.

According to the present invention, a label fixation device is provided. The label fixation device comprises a first reception for temporarily receiving a longitudinal body portion of a medical instrument, and a second reception for arranging a label comprising a pattern or encoding to be fixed to a medical instrument. The first reception and the second reception are movable in relation to each other for providing an abutting contact of a received longitudinal body portion of the medical instrument in the first reception and an arranged label in the second reception. The first reception provides a holding position for a medical instrument, in which holding position the longitudinal body portion is rotatably held around a longitudinal axis of the longitudinal body portion. The second reception provides a supply position for a label, in which supply position a front edge of the label is arranged aligned with the longitudinal axis of the longitudinal body portion.

As an example, also a label fixation device for household use or other fields like material storage, handling and supplying or component/material/equipment tracking is provided. The label fixation device is provided to attach labels to longitudinal portions of a component, equipment, tool, instrument and the like.

The label fixation device comprises a first reception for temporarily receiving a longitudinal body portion of a component, and a second reception for arranging a label comprising a pattern or encoding to be fixed to a component. The first reception and the second reception are movable in relation to each other for providing an abutting contact of a received longitudinal body portion of the component in the first reception and an arranged label in the second reception. The first reception provides a holding position for a component, in which holding position the longitudinal body portion is rotatably held around a longitudinal axis of the longitudinal body portion. The second reception provides a supply position for a label, in which supply position a front edge of the label is arranged aligned with the longitudinal axis of the longitudinal body portion.

It is noted that the following examples and aspects are also provided for household use or other fields like material storage, handling and supplying or material and equipment tracking.

Also, a pattern application system for tracking or identifying components is provided. The system comprises a label printer for printing labels, and a label fixation device according to one of the preceding examples. The label printer is configured to print labels for optical identification or tracking of a component of a plurality of components to be used in a determined environment, like in household, warehouse, storage and material handling. The label fixation device is configured to fix a determined label to a determined component of the plurality of components.

Also, a method for label fixation to a component is proved. The method comprises the following steps:
a) holding a longitudinal body portion of a component in a first reception, wherein the component is hold in a rotatable manner around a longitudinal axis of the longitudinal body portion;
b) arranging a label in a second reception, wherein a front edge of the label is aligned with the longitudinal axis of the longitudinal body portion, and wherein the label is provided with a an adhesive side;
c) moving the longitudinal body portion and the label towards each other such that an abutting contact is provided; and
d) rotating the longitudinal body portion of the component around the longitudinal axis of the longitudinal body portion up-winding the label with the adhesive side onto the longitudinal body portion.

By providing two receptions that are movable to each other, a first stage is provided where the label and the instrument or the like are inserted to the label fixation device. Hence, each of the two can be placed with the required precision in relation to the device and thus automatically also in relation to each other. By providing the two receptions to be movable in relation to each other, both the label and the instrument are brought into touching contact in a precise and desired manner, which allows a fixation of the label onto the instrument with high precision in a repeatable manner. A separate alignment of the label and the instrument to each other by the user of the label fixation device is not required. By arranging the medical instrument and the label in respective receptions, a facilitated placing of the two items is provided. The necessary user attention is reduced to a minimum. Hence, the task of fixing a label to a medical instrument can also be provided by staff members during an operation, even when gloves are worn and sterility is to be observed.

The label contains a pattern that enables at least instrument tracking or identification of the instrument. The tool is easy to use in a sterile environment and enables good alignment of the label with the shaft of the instrument.

The label fixation device can also be referred to as label fixation tool or label fixation apparatus.

The pattern or encoding on the label is provided to be detectable by optical means, such as a camera.

In an example, the label that is fixated to the surgical instrument is used for tracking the position of the instrument in five degrees of freedom, e.g. three positions, two angles.

In a further example, the label that is fixated to the surgical instrument is used for tracking the rotational angle of the instrument around its shaft.

The label may therefore also be referred to as tracking label.

In a still further example, the label that is fixated to the surgical instrument is used for identification of the instrument.

The label may therefore also be referred to as identification label.

The term "medical instrument" relates for example to an instrument used during e.g. a surgical procedure, e.g. to a surgical instrument or surgical device. In an example, the medical instrument is a manually used instrument. In an example, the medical instrument is an instrument to be attached to a further device, like a mechanical instrument or mechanically operated tool.

The word intervention may be used in relation to the work that is performed by a cardiologist or radiologist in a cathlab or vascular lab. These might use small incisions and work inside the body with catheters and the like.

The word surgery may be used in relation to the work that is performed by surgeons in the operation room. These might use open surgery procedures or also minimal invasive procedures.

The body portion may be a longitudinal portion, such as a shaft portion.

The "identification label" relates to a label to be attached to a medical instrument, in order to be able to identify this instrument, for example by optical detection, e.g. by camera detection.

The "tracking label" relates to a label to be attached to a medical instrument, in order to be able to track this instrument, for example by optical detection, e.g. by camera detection.

The holding position is also referred to as first position or first holding position. The supply position is also referred to as second position or second supply position.

The front edge is an edge of a label that is connected to the instrument first when up-winding the label onto the instrument.

According to an example, the first reception provides at least two recesses displaced to each other for providing rotatable hold of a longitudinal body portion of a medical instrument. According to an option, at least one pressing element is provided for urging the longitudinal body portion towards and into the recesses.

According to an example, the two recesses are facing towards the second reception. According to an option, the at least two recesses are having a V-shaped or curved cross section for receiving a longitudinal body portion with a circular cross section. According to a further option, the at least one pressing element comprises a counter recess for providing further support of the rotatable hold of a longitudinal body portion with a circular cross section.

In an example, one specific tool is provided to hold a certain range of device shaft diameters.

In another example, to accommodate a large range of diameters, multiple tools are provided with each being suited for a part of this larger range.

According to an example, for the at least one pressing element, two fixation fingers are provided, which are extending over a front edge of a portion with the first recession.

The fixation fingers are provided to be flexible to be pushed down when inserting the medial instrument. The fixation fingers provide support during the insertion of the medical instrument, before closing the label fixation device. These fixation fingers also allow the operator to release the instrument such that one hand is available to put the label in the second location.

According to an example, the second reception provides a primary label abutting portion, which abutting portion is aligned with a longitudinal axis of the first reception, and which abutting portion provides a stop when inserting a label into the second reception. According to an option, the second reception also provides a secondary label guiding portion, which guiding portion is perpendicular to the primary label abutting portion, and which guiding portion provides a lateral guide when inserting a label into the second reception.

The primary label abutting portion can also be referred to as label forward edge. The secondary label guiding portion can also be referred to as label bottom edge.

According to an example, the primary label abutting portion comprises at least one movable frontal edge that is projecting from the second reception to provide the abutting for the label when arranged in the second reception.

In an option, the at least one movable frontal edge is retractable to project less to allow a touching of the body portion with the label.

For example, the at least one movable frontal edge comprises two or more projections as frontal edge sections.

In an example, the at least one movable frontal edge is attached to a leaf spring allowing vertical movement of the least one movable frontal edge.

According to an example, the first reception is provided by a first body part of the label fixation device. The second reception is provided by a second body part of the label fixation device. The first body part and the second body part are movable in relation to each other between an insertion position and a label-fixation position. In the insertion position, the first and second receptions are spaced apart, and in the label-fixation position, the first and second receptions are closer to each other to provide the abutting contact.

In the insertion position, the first reception is provided with a larger distance towards the second reception compared to the label-fixation position.

In an example, the first body part and the second body part are connected by a hinge. For example, an elastic member is provided biasing the hinge towards the insertion position.

The first and second part are forming a clamp-like arrangement that can be opened manually by pressing on two portions extending beyond the hinge connection. The clamp-like arrangement provides a clip-type arrangement.

When the label and the instrument are in place, the tool can be closed and the longitudinal body portion is approaching the label edge. The longitudinal body portion will hit the frontal edges and push them down until the longitudinal body portion touches the label.

In an example, the first body part and the second body part are providing grip portions on the outside to manually urge the two parts against each other to hold a received longitudinal body portion of a medical instrument.

According to an example, in the label-fixation position, an inserted longitudinal body portion of a medical instrument is urged against an arranged label, such that an adhesive provided on one side of the label sticks to the body portion.

According to an example, in the label-fixation position, an inserted longitudinal body portion of a medical instrument is manually rotatable around the longitudinal axis to provide a complete winding-up of the label onto the body portion.

The winding-up is also referred to as rolling-up the label. By winding the label around the body portion, a continuous labelling around the body portion is provided.

According to an example, at least one label is provided that comprises a graphic pattern with features allowing improved tracking of the instrument, or detecting of an instrument's rotation angle and/or instrument identifying.

In an option, at least one label is provided that comprises a number of alternating parallel stripes of contrary brightness and/or color, and wherein the stripes are to be aligned around the body portion of a medical instrument such that they provide a number of continuous rings around the body portion.

According to an example, the device is provided as a handhold device.

In an example, the device is made basically from plastic. The label fixation device may be provided to be a disposable device. For example, the device is disposed after an intervention like a surgical operation together with used instruments. Of course, disposal may include providing the device to a recycling process.

In an example, the device is sterilized, when made from an appropriate material, and re-used.

According to the present invention, also a pattern application system for tracking or identifying medical instruments is provided. The system comprises a label printer for printing labels, and a label fixation device according to one of the preceding examples. The label printer is configured to print labels for optical identification or tracking of a medical instrument of a plurality of medical instruments to be used during a medical intervention, like a surgical procedure or other intervention. The label fixation device is configured to fix a determined label to a determined medical instrument of the plurality of medical instruments.

For example, the label printer provides sterile labels.

In another option, a number of labels will be supplied together with the fixation tool in a sterile packaging, e.g. to be used for a specific patient.

In an example, a surgical navigation system is provided, in which both the patient and the device are tracked using small optical cameras. For example, the optical tracking provides primarily the position of patient markers and related to this patient the device position.

According to an example, a plurality of medical instruments is provided, for example used by the surgeon, and wherein at least a part of the medical instruments are identifiable and can be tracked by labels fixed by the label fixation device.

In an example, the tools are disposable surgical tools or surgical instruments.

According to the present invention, also a method for label fixation to a medical instrument is proved. The method comprises the following steps:
a) holding a longitudinal body portion of a medical instrument in a first reception, wherein the medical instrument is hold in a rotatable manner around a longitudinal axis of the longitudinal body portion;
b) arranging a label in a second reception, wherein a front edge of the label is aligned with the longitudinal axis of the longitudinal body portion, and wherein the label is provided with a an adhesive side;
c) moving the longitudinal body portion and the label towards each other such that an abutting contact is provided; and
d) rotating the longitudinal body portion of the medical instrument around the longitudinal axis of the longitudinal body portion up-winding the label with the adhesive side onto the longitudinal body portion.

According to an example, in step b), the label is provided with an uncovered region of the adhesive side next to the abutting edge and with a covered region of the adhesive side distal to the net to the abutting edge. In step d), a first sub-step d1) of rotating is provided for an up-winding of a first part of the label. In an intermediate step e), a cover is removed from the covered side. A second sub-step d2) of rotating is provided for an up-winding of a rest part of the label.

According to an aspect, a label fixation device is provided, which provides separate placing of label and instrument, each achieved by providing a predetermined positioning of instrument and label in relation to the device. For the placement, two different receptions are arranged. By moving the receptions in relation to each other, both items, label and instrument, are brought into contact for attaching the label to the instrument. The label is held in alignment to the device and upon an initial touching, the rest of the label can be attached and thus applied to the instrument by an adhesive or adhesive layer arranged for example on the label.

The label is provided as a printable sheet or foil, for example made from a plastic material. The labels can also be provided with one or more patterns already printed in the factory such that more than one instrument can be identified and tracked.

In an option, adhesive is provided on the instrument (e.g. as an adhesive layer) before placing the instrument in the reception of the labelling apparatus (i.e. the label fixation device). The label can then be provided with or without adhesive.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically illustrates an example of a label fixation device.
Fig. 2 shows a perspective view of a further example of a label fixation device.
Fig. 3 shows the label fixation device of Fig. 2 in an opened state for inserting a longitudinal body portion of a medical instrument and a label.
Fig. 4A shows a further example of label fixation device the in a closed state, and Fig. 4B in a partly opened state and Fig. 4C in an opened state.
Fig. 5A illustrates an initial situation showing a medical instrument, a label and a label fixation device.
Fig. 5B shows the label with only a part of a protection peeled off from an adhesive side.
Fig. 5C shows the medical instrument inserted into the label fixation device.
Fig. 5D shows the label inserted into the label fixation device.
Fig. 5E shows the label in a touching contact with the medical instrument.
Fig. 5F shows the label partly wound onto the medical instrument.
Fig. 5G shows the label nearly completely wound-up onto the medical instrument.
Fig. 5H shows the label completely wrapped around the medical instrument and the label fixation device in an opened state where the medical instrument is ready to be taken out as labelled instrument.
Fig. 6 schematically illustrates an example of a pattern application system for tracking or identifying medical instruments with a label printer for printing labels and a label fixation device.
Fig. 7 illustrates basic steps of an example of a method for label fixation to a medical instrument.

### DETAILED DESCRIPTION OF EMBODIMENTS

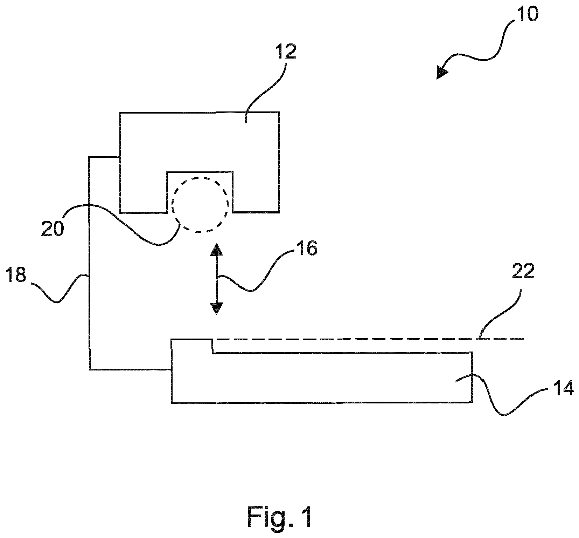

Fig. 1 schematically illustrates an example of a label fixation device 10. The label fixation device 10 comprises a first reception 12 for temporarily receiving a longitudinal body portion of a medical instrument. The label fixation device 10 also comprises a second reception 14 for arranging a label to be fixed to a medical instrument. The first reception 12 and the second reception 14 are movable (indicated with a double arrow 16) in relation to each other for providing an abutting contact of a received longitudinal body portion of the medical instrument in the first reception 12 and an arranged label in the second reception 14. A connection line 18 indicates a mechanical connection between the first reception 12 and the second reception 14. This mechanical connection has to realize proper alignment of reception 12 and reception 14 during the movement 16.

The first reception 12 provides a holding position for a medical instrument, indicated with a dotted circle 20. In the holding position, the longitudinal body portion is rotatably held around a longitudinal axis of the longitudinal body portion.

The second reception 14 provides a supply position for a label, indicated with a dotted line 22. In the supply position, a front edge of the label is arranged aligned with the longitudinal axis of the longitudinal body portion.

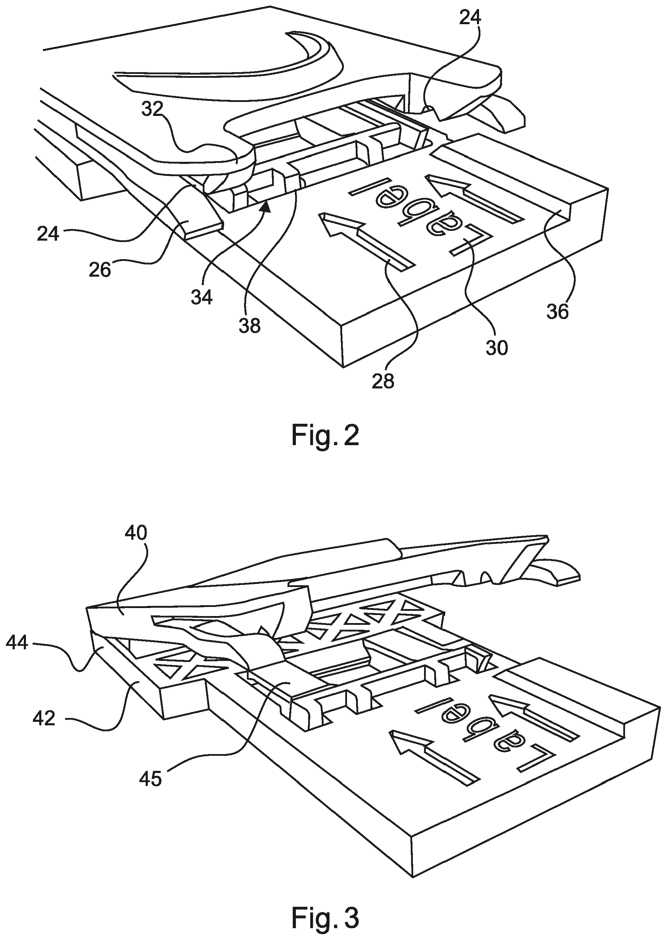

Fig. 2 shows a perspective view of a further example of the label fixation device 10. The first reception 12 provides at least two recesses 24 displaced to each other for providing rotatable hold of a longitudinal body portion of a medical instrument (not shown).

The two recesses 24 are facing towards the second reception 14. In an option, not shown in detail, the at least two recesses 24 are having a V-shaped or curved cross section for receiving a longitudinal body portion with a circular cross section. This provides a precise fixation while still allowing a rotational movement of the longitudinal body portion, e.g. a shaft.

As an option, at least one pressing element 26 is provided for urging the longitudinal body portion towards and into the recesses. As can be seen, the example shows two such pressing elements 26 as an option.

In a further option, the at least one pressing element 26 comprises a counter recess for providing further support of the rotatable hold of a longitudinal body portion with a circular cross section.

The second reception 14 is shown with two arrows 28 indicating an insertion direction for the label. Further a text, such as "Label" may provide further guide for operating the device.

As indicated, in the example of Fig. 2, the pressing elements 26 are provided as two fixation fingers, which are extending over a front edge 32 of a portion with the first recession.
In an option, the second reception 14 provides a primary label abutting portion 34, which abutting portion 34 is aligned with a longitudinal axis of the first reception 12. The abutting portion 34 provides a stop when inserting a label into the second reception 14.

In another option, the second reception 14 also provides a secondary label guiding portion 36, which is perpendicular to the primary label abutting portion 34, and which guiding portion 36 provides a lateral guide when inserting a label into the second reception 14. For a proper alignment of the label, which label is usually based on a rectangular layout, the primary label abutting portion is perpendicular to the secondary label abutting portion. In case of an otherwise inclined arrangement of the primary and the label abutting portions, the layout of the label, i.e. the pattern arranged in the label, would have to take this into account.

The purpose of the label abutting portions is to provide the label in an exact and reproducible manner in relation to the instrument, i.e. the portion of the instrument, onto which the label is to be fixedly placed.

As indicated, in an example, the label is stopped by two vertical edges. These have to make sure that the label is aligned with the instrument. Small grooves are shown that support to achieve the contact between the label and the instrument over a large area when pressing both against each other.

In one example, shown as an option, the primary label abutting portion 34 comprises at least one movable frontal edge 38, for example, two, three or four or more edge portions, that is (are) projecting from the second reception 14 to provide the abutting for the label when arranged in the second reception 14.

As an example, in Fig. 2 there are four edges 38. The two edges of these in the middle will make contact with the label and are intended as the end stops. The two outer edges are placed in positions that exceed the label dimension and will not make contact with the label. Their upper sides are more elevated than the upper sides of the inner edges. When the shaft is pushed down, the inner edges also move down. Their upper side will move down below the label itself, because the upper sides of the outer edges are more elevated). This ensures that the label can be rolled-up without being hindered by the inner edges that have been serving as an end-stop before.

For example, the at least one movable frontal edge 38 is retractable to project less to allow a touching of the body portion with the label (see also below).

Fig. 3 shows the label fixation device 10 of Fig. 2 in an opened state for inserting a longitudinal body portion of a medical instrument and a label. The first reception 12 is provided by a first body part 40 of the label fixation device. The second reception 14 is provided by a second body part 42 of the label fixation device. The first body part 40 and the second body part 42 are movable in relation to each other between an insertion position *P_{I}* and a label-fixation position *P_{F}* (shown in Fig. 2). In the insertion position, the first and second receptions are spaced apart, and in the label-fixation position, the first and second receptions are closer to each other to provide the abutting contact.

As also shown, horizontal leaf springs 45 are provided which holds the at least one movable frontal edge allowing vertical movement of the least one movable frontal edge.

In the label-fixation position, an inserted longitudinal body portion of a medical instrument is urged against an arranged label, such that an adhesive provided on one side of the label sticks to the body portion.

In the label-fixation position, an inserted longitudinal body portion of a medical instrument is manually rotatable around the longitudinal axis to provide a complete winding-up of the label onto the body portion.

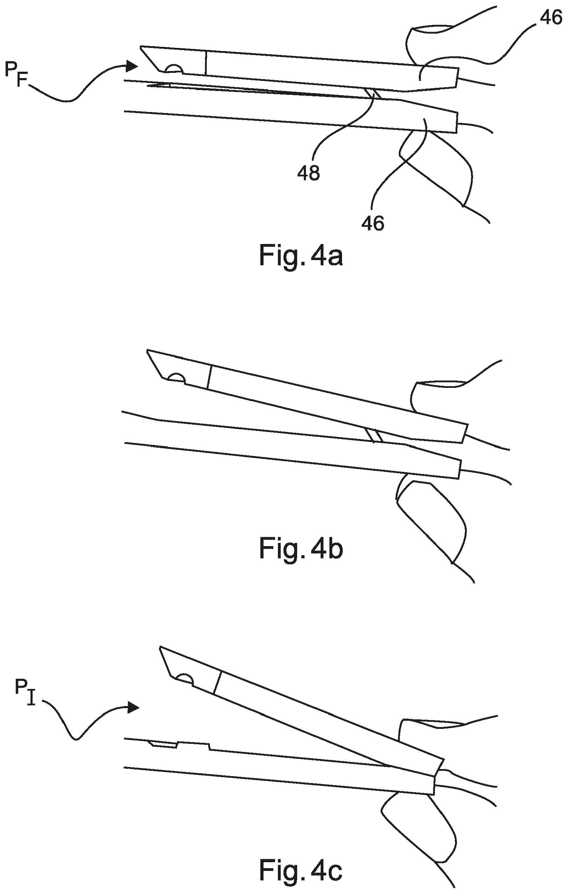

Fig. 4A shows a further example of the label fixation device 10 in a closed state providing the label-fixation position *P_{F}*. The first and second part are connected and biased to urge against each other. Back endings 46 may overlap a hinge 48 such that by fingers acting here as pressure force, the device can be opened.

Fig. 4B shows the label fixation device 10 in a partly opened state.

Fig. 4C shows the label fixation device 10 in an opened state providing the insertion position *P_{I}.*

In the following, an exemplary sequence of applying a label to a device is described.

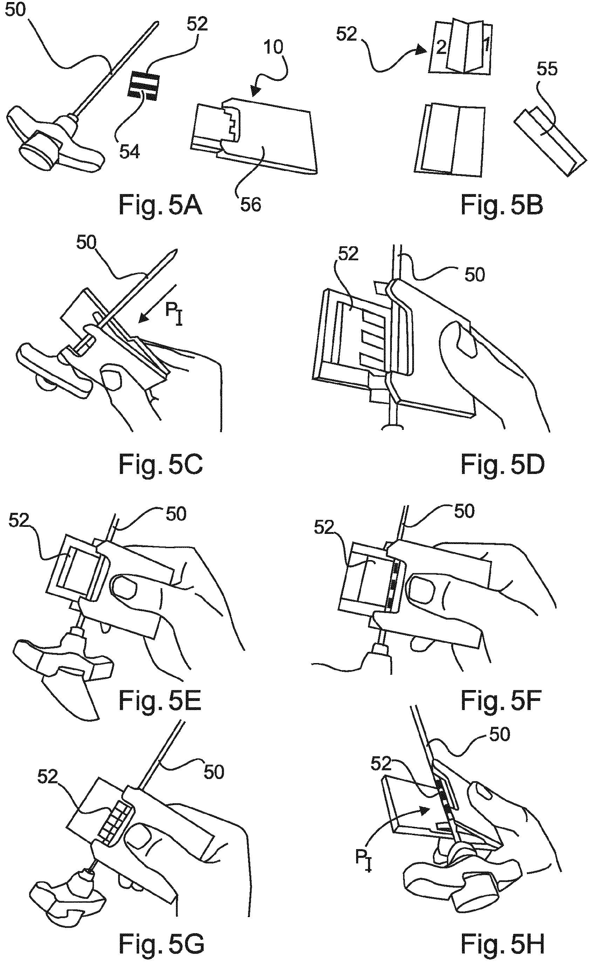

Fig. 5A illustrates an initial situation showing a medical instrument 50, a label 52 and an example of the label fixation device 10. The label 52 comprises a number of alternating parallel stripes 54 of contrary brightness and/or color. The stripes are to be aligned around the body portion of the medical instrument 50 such that they provide a number of continuous rings around the body portion. All parts are ready and sterile.

Fig. 5B shows the label 52 with only a part 55 of a protection peeled off from an adhesive side. The half-peeled label is now ready for insertion. In other words, a first part of the paper cover is removed.

The label fixation device 10 is shown as a handhold device 56.

Fig. 5C shows the medical instrument 50 inserted into the label fixation device 10. The user presses on the back ends and thus opens the device to achieve the inserting position *P_{I}.* The device in the tool is put in the proper position.

Fig. 5D shows the label 52 inserted into the label fixation device 10. The medical instrument 50 is held by the pressing elements that are provided as two fixation fingers. The label fixation device 10 can now be closed. The label is positioned against both edges.

Fig. 5E shows the label 52 in a touching contact with the medical instrument 50. In addition to a biasing, the user can press the device. Due to the secure but rotatable hold the user can now start to rotate the tool such that the label starts winding-up onto the device. The tool is closed and the shaft is pushed against the label. (At this time, the vertical edges 38 have been pushed down below the label such that they will not hinder the label from passing the edges when the shaft is rotated to wind-up the label.)

Fig. 5F shows the label 52 partly wound onto the medical instrument 50. The device is rotated and paper number 2 is removed. The medical instrument is rotated further to apply the whole label. The device can then be removed from the tool.

Fig. 5G shows the label 52 nearly completely wound-up onto the medical instrument.

Fig. 5H shows the label 52 completely wrapped around the medical instrument 50 and the label fixation device 10 in an opened state where the medical instrument is ready to be taken out as labelled instrument.

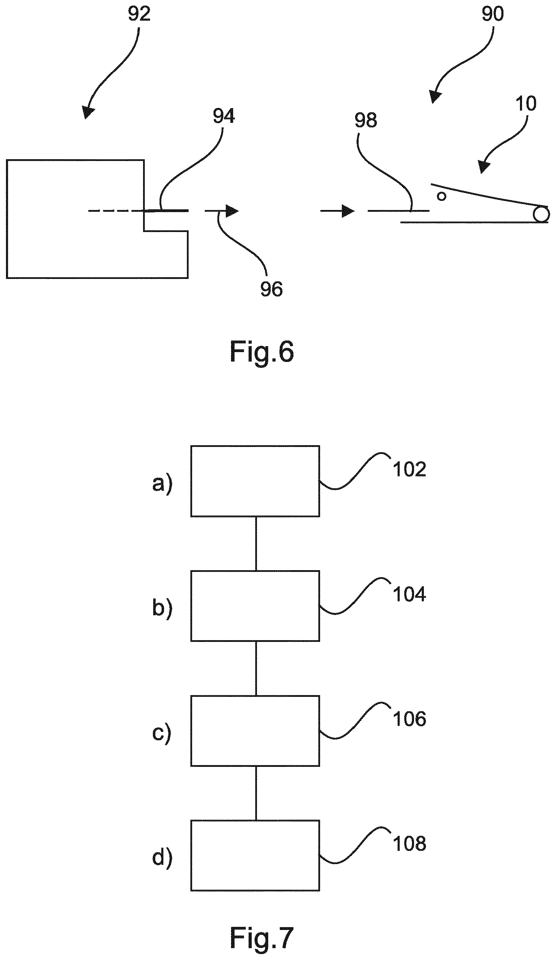

Fig. 6 schematically illustrates an example of a pattern application system 90 for tracking or identifying medical instruments with a label printer 92 for printing labels and an example of the label fixation device 10 according to one of the above examples. The label printer 92 is configured to print labels, such as identification labels or tracking labels, for optical identification or tracking of a medical instrument of a plurality of medical instruments to be used during an intervention like a surgical procedure. For example, the label printer 92 provides a number of labels 94 adapted to the respective device to which the label is to be attached. The labels are provided (as indicated by arrow 96) to the label fixation device 10, where an arrow 98 indicates the insertion into the second reception. The label fixation device is configured to fix a determined label to a determined medical instrument of the plurality of medical instruments.

In an example, not further illustrated, a plurality of medical instruments is provided and at least a part of the medical instruments are identifiable or trackable by labels fixed by the label fixation device 10.

In an example, pre-manufactured labels are provided for different instruments that are supplied in sterile packages, together with the tool. In another example, the labels are provided without the tool.

Fig. 7 illustrates basic steps of an example of a method 100 for label fixation to a medical instrument. The method 100 comprises the following steps.
- In a first step 102, also referred to as step a), a longitudinal body portion of a medical instrument is hold in a first reception. The medical instrument is held in a rotatable manner around a longitudinal axis of the longitudinal body portion.
- In a second step 104, also referred to as step b), a label is arranged in a second reception, wherein a front edge of the label is aligned with the longitudinal axis of the longitudinal body portion, and wherein the label is provided with an adhesive side.
- In a third step 106, also referred to as step c), (106) the longitudinal body portion and the label are moved towards each other such that an abutting contact is provided. and
- In a fourth step 108, also referred to as step d), the longitudinal body portion of the medical instrument rotates around the longitudinal axis of the longitudinal body portion up-winding the label with the adhesive side onto the longitudinal body portion while the operator is pressing the shaft against the label.

In an example, in step b), the label is provided with an uncovered region of the adhesive side next to the abutting edge and with a covered region of the adhesive side distal to the abutting edge. In step d), a first sub-step d1) of rotating is provided for an up-winding of a first part of the label. In an intermediate step e), a cover is removed from the covered side. Further, a second sub-step d2) of rotating is provided for an up-winding of a rest part of the label.

In an aspect, increasingly more surgical procedures are performed minimally invasive. Solutions have been developed to improve the ease of use and the obtainable accuracy for the surgeons.

In an example, one camera is provided that is used to track the positions of devices by their labels.

In an option, the label is provided to reflect infrared or ultraviolet light, i.e. invisible light, for optical tracking.

According to an aspect, for device tracking a specific pattern is applied around the shaft of a device. For example, a sticky label is applied to the shaft. To facilitate the fixation of the label, for example in a sterile environment for a person wearing gloves, a tool is provided that facilitates the labelling process.

In an example, elastic fingers push the device shaft in the V-shaped grooves. The forward and bottom edges are used to align the label in the tool. In case of a rotation point at the back side of the tool that allows to open or close the tool. This rotation point should have very little or no backlash (play) such that the alignment of device shaft and label edges are maintained. The forward edges are mounted on two horizontal leaf springs such that they can be pushed down. When the label and device are in place, the tool can be closed and the device shaft is approaching the label edge. The shaft will hit the frontal edges and push them down until the shaft touches the label.

The label fixation tool is intended to apply a label with a marker pattern on the device. The label must be applied with the pattern perpendicular to the shaft of the device, otherwise the pattern lines at the edge of the applied label will not match. To apply such a label perpendicular in a sterile environment for a person wearing gloves is cumbersome if not impossible. The tool facilitates this task.

In an example, the tool is 3D printed out of strong and flexible plastic. In further examples, other material is used. The tool can also be assembled from individual parts.

For operation, the device is placed in the tool and can be released later. The tool will hold the device in place. This way one hand is free to pick up the label. The label can be placed in the tool against the bottom edge and the edge underneath the shaft of the device. In this way, the label is aligned with respect to the device and the pattern lines will be perpendicular to the shaft of the device.

The hand that is holding the tool can be used to push the shaft against the label. Pressure can be maintained while the device is rotated such that the whole label is firmly attached to the shaft of the device.

After turning the device slightly inside the tool, the second paper covering the adhesive part of the label can be removed easily by releasing the device. The device will remain steady inside the tool. When the second paper is removed, the device can be turned further until the complete label is applied.

The tool is further optimized for ease-of-use:
- A thumb-like shape indicates where to push (best to push in the middle of the tool rather than close to the edge).
- An indication can be added that indicates how and where to position the label (with the label pattern against the surface of the tool and the label edges against the tool edges).

Some specific advantages of the label and tool combination are also provided:
- The tool is suited to fit a certain range of shaft diameters. For example, one tool could be used for any shaft diameter in the range of 2,5 ... 4 mm.

The combination of tool and label is suited to apply markers on any device that has a cylindrical part in its shaft. The surgeon is capable of using his preferred devices.

Depending on the material properties, the tool can be supplied in a sterile packaging, e.g. together with some labels or it can be sterilized by the hospital staff and be reused for multiple procedures.

As an effect, a nurse or surgeon in a sterile setting can quickly apply a label accurately to a surgical instrument, while working in a sterile environment with gloves.

In an example, the tool is provided for assisting in fixation of a marker label to an instrument shaft or another suitable portion of the instrument. The device shaft is placed into a reception. Elastic fingers are provided that support this by pushing, i.e. urging, the device shaft into the reception. For example, the reception is provided by V-shaped grooves. A label is arranged which is positioned by forward and bottom edges for the label to align the label in the tool. The tool is having two parts, connected to each other via a rotation point, which allows to open or close the tool. The rotation point should have very little or no play such that the alignment of the device shaft and the label edges is maintained. The forward edges for the label placing are mounted on two horizontal leaf springs such that they can be pushed down. When the label and the device are in place, the tool is closed and the device shaft is approaching the label edge. The shaft will hit the frontal edges and push them down until the shaft touches the label.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A label fixation device (10), comprising:
- a first reception (12) for temporarily receiving a longitudinal body portion of a medical instrument; and
- a second reception (14) for arranging a label to be fixed to a medical instrument;
wherein the first reception and the second reception are movable (16) in relation to each other for providing an abutting contact of a received longitudinal body portion of the medical instrument in the first reception and an arranged label in the second reception; and
wherein the first reception provides a holding position for a medical instrument, in which holding position the longitudinal body portion is rotatably held around a longitudinal axis of the longitudinal body portion; and
wherein the second reception provides a supply position for a label, in which supply position a front edge of the label is arranged aligned with the longitudinal axis of the longitudinal body portion.

2. Device according to claim 1, wherein the first reception provides at least two recesses (24) displaced to each other for providing rotatable hold of a longitudinal body portion of a medical instrument; and
wherein, preferably, at least one pressing element (26) is provided for urging the longitudinal body portion towards and into the recesses.

3. Device according to claim 2, wherein the two recesses are facing towards the second reception;
wherein, preferably, the at least two recesses are having a V-shaped or curved cross section for receiving a longitudinal body portion with a circular cross section; and
wherein, further preferably, the at least one pressing element comprises a counter recess for providing further support of the rotatable hold of a longitudinal body portion with a circular cross section.

4. Device according to one of the preceding claims, wherein, for the at least one pressing element, two fixation fingers are provided, which are extending over a front edge (32) of a portion with the first recession.

5. Device according to one of the preceding claims, wherein the second reception provides a primary label abutting portion (34), which abutting portion is aligned with a longitudinal axis of the first reception, and which abutting portion provides a stop when inserting a label into the second reception; and
wherein, preferably, the second reception also provides a secondary label guiding portion (36), which guiding portion is perpendicular to the primary label abutting portion, and which guiding portion provides a lateral guide when inserting a label into the second reception.

6. Device according to one of the preceding claims, wherein the primary label abutting portion comprises at least one movable frontal edge (38) that is projecting from the second reception to provide the abutting for the label when arranged in the second reception; and
wherein, preferably, the at least one movable frontal edge is retractable to project less to allow a touching of the body portion with the label.

7. Device according to one of the preceding claims, wherein the first reception is provided by a first body part (40) of the label fixation device; and the second reception is provided by a second body part (42) of the label fixation device; and
wherein the first body part and the second body part are movable in relation to each other between an insertion position (P_{I}) and a label-fixation position (P_{F}); wherein in the insertion position, the first and second receptions are spaced apart, and in the label-fixation position, the first and second receptions are closer to each other to provide the abutting contact.

8. Device according to one of the preceding claims, wherein in the label-fixation position, an inserted longitudinal body portion of a medical instrument is urged against an arranged label, such that an adhesive provided on one side of the label sticks to the body portion.

9. Device according to one of the preceding claims, wherein, in the label-fixation position, an inserted longitudinal body portion of a medical instrument is manually rotatable around the longitudinal axis to provide a complete winding-up of the label onto the body portion.

10. Device according to one of the preceding claims, wherein at least one label (52) is provided that comprises a graphic pattern with features allowing improved tracking of the instrument, or detecting of an instrument's rotation angle and/or instrument identifying; and
wherein, preferably, the label comprises a number of alternating parallel stripes (54) of contrary brightness and/or color, and wherein the stripes are to be aligned around the body portion of a medical instrument such that they provide a number of continuous rings around the body portion.

11. Device according to one of the preceding claims, provided as a handhold device (56).

12. A pattern application system (90) for tracking or identifying medical instruments, the system comprising:
- a label printer (92) for printing labels; and
- a label fixation device (10) according to one of the preceding claims;
wherein the label printer is configured to print labels for optical identification or tracking of a medical instrument of a plurality of medical instruments to be used during a medical intervention; and
wherein the label fixation device is configured to fix a determined label to a determined medical instrument of the plurality of medical instruments.

13. System according to claim 12, wherein a plurality of medical instruments is provided and wherein at least a part of the medical instruments are identifiable and/or trackable by labels fixed by the label fixation device.

14. A method (100) for label fixation to a medical instrument, the method comprising the following steps:
a) holding (102) a longitudinal body portion of a medical instrument in a first reception, wherein the medical instrument is hold in a rotatable manner around a longitudinal axis of the longitudinal body portion;
b) arranging (104) a label in a second reception, wherein a front edge of the label is aligned with the longitudinal axis of the longitudinal body portion, and wherein the label is provided with a an adhesive side;
c) moving (106) the longitudinal body portion and the label towards each other such that an abutting contact is provided; and
d) rotating (108) the longitudinal body portion of the medical instrument around the longitudinal axis of the longitudinal body portion up-winding the label with the adhesive side onto the longitudinal body portion.

15. Method according to claim 14, wherein in step b) the label is provided with an uncovered region of the adhesive side next to the abutting edge and with a covered region of the adhesive side distal to the abutting edge; and wherein in step d), a first sub-step d1) of rotating is provided for an up-winding of a first part of the label; and in an intermediate step e), a cover is removed from the covered side; and a second sub-step d2) of rotating is provided for an up-winding of a rest part of the of the label.
